**Europäisches Patentamt**

**European Patent Office**

**Office européen des brevets**

(19)

(11) Veröffentlichungsnummer: **0 218 124**

**B1**

(12) # EUROPÄISCHE PATENTSCHRIFT

(45) Veröffentlichungstag der Patentschrift:
23.11.88

(21) Anmeldenummer: **86112815.5**

(22) Anmeldetag: **17.09.86**

(51) Int. Cl.⁴: **B 01 J 37/02** //
B01J23/50, C07D301/10,
C07C45/38, C07C47/21

(54) **Trägerkatalysator, Verfahren zu seiner Herstellung und seine Verwendung.**

(30) Priorität: **20.09.85 DE 3533573**

(43) Veröffentlichungstag der Anmeldung:
**15.04.87 Patentblatt 87/16**

(45) Bekanntmachung des Hinweises auf die Patenterteilung:
**23.11.88 Patentblatt 88/47**

(84) Benannte Vertragsstaaten:
**CH DE FR GB IT LI NL**

(56) Entgegenhaltungen:
EP-A-0 033 202

**JOURNAL OF APPLIED PHYSICS, Band 47, Nr. 9,
September 1976, Seiten 3799-3803, American
Institute of Physics, US; R.C. BAETZOLD: "Study
of evaporated metal nuclei by Auger spectroscopy"**

(73) Patentinhaber: **BASF Aktiengesellschaft, Carl-Bosch- Strasse 38, D-6700 Ludwigshafen (DE)**

(72) Erfinder: **Bittler, Knut, Dr., Kardinal- Wendel-Strasse 54, D-6720 Speyer (DE)**
Erfinder: **Broecker, Franz Josef, Dr., Schwanthaler Allee 20, D-6700 Ludwigshafen (DE)**
Erfinder: **Aquila, Werner, Dr., Meissener Weg 35, D-6800 Mannheim 31 (DE)**
Erfinder: **Scheuring, Hubertus, Winklerstrasse 2, D-6710 Frankenthal (DE)**

**Beschreibung**

Die Erfindung betrifft einen Trägerkatalysator in Form eines mit einer Silberschicht als Aktivkomponente versehenen Trägermaterials, insbesondere eines netz-, gewebe- oder folienartigen Trägermaterials, ein Verfahren zur Herstellung des Trägerkatalysators und dessen Verwendung bei der oxidativen Dehydrierung organischer Verbindungen, insbesondere der Herstellung von 3-Methyl-buten-1-alen durch oxidative Dehydrierung von 3-Methyl-buten-1-olen, sowie dessen Verwendung bei der Oxidation ungesättigter organischer Verbindungen.

Im Stand der Technik sind eine Vielzahl silberhaltiger Trägerkatalysatoren bekannt, die für die verschiedensten Zwecke eingesetzt werden. So beschreibt beispielsweise die DE-A-1 793 348 einen silberhaltigen Trägerkatalysator, dessen Träger aus einem einfach oder mehrfach gerollten Metallgewebe besteht, auf welches Silberoxid, gegebenenfalls mehrmals, in Form einer wäßrigen Suspension aufgebracht und vor jedem weiteren Auftrag in bekannter Weise reduziert wurde. Dieser Katalysator wird zur Herstellung von Ethylenoxid durch Oxidation von Ethylen mit Sauerstoff empfohlen.

Die DE-A-2 211 421 betrifft die Herstellung von Hydroxycitronellal aus Hydroxycitronellol in Gegenwart eines Silberkatalysators, der zweckmäßig als Überzug auf einer Metallgaze aufgebracht ist. Es handelt sich hierbei im wesentlichen um denselben Katalysator, wie er in der DE-A-1 793 348 beschrieben ist.

Soweit nach dem Stand der Technik Silberkatalysatoren zur oxidativen Dehydrierung, beispielsweise von 3-Methyl-buten-1-olen verwendet werden, liegen diese als Schüttungen von Silber- und Kupfergranulat vor, wobei man bereits erhebliche Anstrengungen unternommen hat, eine gleichmäßige Durchströmung der Reaktionszone zu erreichen.

So beschreibt beispielsweise die DE-A-2 715 209 ein Verfahren, bei dem man mit Silber- und/oder Kupfergranulatschichten arbeitet, wobei die gesamte Katalysatorschicht aus einzelnen Schüttungen unterschiedlicher Korngröße aufgebaut ist. Auch bei diesem Verfahren treten in der Katalysatorschicht unregelmäßige Verweilzeiten und dadurch örtliche Überhitzungen auf, die zum Sintern und Zusammenbacken des Katalysators führen. Das Sintern und Verbacken der Silbergranulatkörner führt zur Kanal- und Rißbildung innerhalb der Schicht und dadurch zu schneller Abnahme von Umsatz und Selektivität.

In der US-A-2 042 220 ist die Oxidation ungesättigter Alkohole mit Sauerstoff im Überschuß bei 200 bis 550° C in Gegenwart von Metallkatalysatoren, beispielsweise Kupfer- und Silberkatalysatoren zu ungesättigten Aldehyden beschrieben. Die Katalysatoren können Legierungen, Metallverbindungen oder elementares Metall sein. Bevorzugt werden aktivierte Katalysatoren, wobei die Aktivierung durch eine Amalgamierung des Metalls mit anschließender Temperung erfolgt. Die Herstellung von Kupfer- und Silberkatalysatoren in den beschriebenen Beispielen erfolgt einmal durch Reduktion von Kupferoxiddraht bei 300° C mit Wasserstoff und zum anderen durch Amalgamierung und anschließendes Erhitzen von Silberdraht.

Was die Umsetzung von 3-Methyl-buten-1-olen zu 3-Methylbuten-1-alen anbetrifft, werden nach der DE-A-2 041 976 erhebliche Mengen an Isovaleraldehyd und Isopren gebildet, was zu einer verringerten Ausbeute an Wertprodukt führt. Bei Zumischen von Sauerstoff erhält man zunehmende Mengen an Dimethylacrylsäure und damit ebenfalls Ausbeuteverluste.

Nachteilig an den bisher bekannten Trägerkatalysatoren in Form eines mit einer Silberschicht als Aktivkomponente versehenen netz-, gewebe- oder folienartigen Trägermaterials ist insbesondere der Umstand, daß Katalysatoraktivität, Selektivität, sowie Standzeit stark zu wünschen übrig lassen.

Der Erfindung liegt die Aufgabe zugrunde, einen Trägerkatalysator der eingangs genannten Art bereitzustellen, der die Nachteile des Standes der Technik nicht aufweist und bei hoher Aktivität, Selektivität und Standzeit die Durchführung der verschiedensten Reaktionen, insbesondere oxidative Dehydrierungen, bevorzugt die Herstellung von 3-Methyl-buten-1-alen durch oxidative Dehydrierung von 3-Methyl-buten-1-olen, erlaubt.

Diese Aufgabe wird gelöst mit einem Trägerkatalysator der genannten Art, der dadurch gekennzeichnet ist, daß die Silberschicht polykristallin ist und durch Bedampfen des Trägermaterials mit metallischem Silber und anschließende thermische Behandlung bei 200 bis 800° C gebildet wurde.

Überraschenderweise hat sich gezeigt, daß durch Kombination der Maßnahmen des Bedampfens des Trägermaterials und anschließende thermische Behandlung des Trägerkatalysators die aufgedampfte Silberschicht eine außergewöhnlich Aktivierung erfährt, wodurch man einen hochaktiven Trägerkatalysator erhält.

Nach einer bevorzugten Ausführungsform erfolgt die thermische Behandlung unter Reaktionsbedingungen. Der Begriff "unter Reaktionsbedingungen" bedeutet, daß - je nach speziellem Einsatz des Katalysators - die thermische Behandlung zu Beginn der eigentlichen, jeweiligen Reaktion im Reaktionsbereich erfolgt. Besonders zweckmäßig führt man die thermische Behandlung bei 300 bis 500° C durch.

Das Trägermaterial kann jegliche gewünschte Form aufweisen. Insbesondere kann es netz-, gewebe- oder folienartig sein. Besonders zweckmäßig ist das Trägermaterial netzförmig. Als Trägermaterial läßt sich z. B. Drahtgewebe aus den unterschiedlichsten webbaren Metalldrähten verwenden. Hierbei kommen Eisen, Federstahl, Kupfer, Messing, Phosphorbronze, Reinnickel, Monel-Metall, Aluminium, Silber, Neusilber, Nickel, Chromnickel, Chromstahl, nicht rostende, säurebeständige und hochhitzebeständige Chromnickelstähle, sowie Titan in Betracht. Besonders bevorzugt ist ein Trägermaterial aus Chrom/Aluminium-Edelstahlgewebe oder -netz. So kann man beispielsweise ein unter der Handelsbezeichnung Kanthal® bekanntes Material, beispielsweise mit den Werkstoffnummern 1.4765 und insbesondere 1.4767, verwenden.

Die genannten Chrom/Aluminium-Edelstahlgewebe können beispielsweise ungefähr 20 bis 30 Gew.-%, insbesonderes 23 % Chrom, 3 bis 8 Gew. %, insbesondere 6 % Aluminium, 0 bis 4 Gew.-%, insbesondere 2 % Kobalt, geringe Mengen an Kohlenstoff, z. B. 0,06 %, sowie Eisen als Differenz, enthalten. Eine andere geeignete Zusammensetzung enthält ungefähr 20 % Chrom, 4,5 % Aluminium, 0,5 % Kobalt, mit Eisen als Differenz.

Als Trägermaterial lassen sich auch synthetische Drähte aus Kunststoffen einsetzen. Hierbei kommen in Betracht Polyamide, Polyester, Polyethylen, Polyvinylverbindungen, Polypropylen, Polytetrafluorethylen und ähnliche Träger. Man kann insbesondere nicht rostende und hochhitzebeständige Stähle, sowie Gewebe aus hitzebeständigen anorganischen Fasern, beispielsweise Keramikfasern, wählen. Gewebe unterschiedlicher Webart, beispielsweise glatte Gewebe, Körpergewebe, Tressengewebe, Fünfschaft-Atlas-Gewebe und andere Spezialbindungsgewebe kommen in Betracht. Auch Formlinge aus Drahtgestrick, beispielsweise Raschigringe, oder auch andere Trägermaterialien können eingesetzt werden. Prinzipiell kann man auch α-Aluminiumoxidträger beliebiger Form einsetzen.

Gegenstand der Erfindung ist auch ein Verfahren zur Herstellung des Trägerkatalysators durch Aufbringen einer Silberschicht auf das Trägermaterial, wobei das Verfahren dadurch gekennzeichnet ist, daß man die Silberschicht auf das Trägermaterial aufdampft und anschließend bei 200 bis 800°C eine thermische Behandlung durchführt. Das aufzubringende Silber wird besonders zweckmäßig im Vakuum bei $10^{-1}$ bis $10^{-12}$ bar, insbesondere $10^{-6}$ bis $10^{-10}$ bar, verdampft und auf das Trägermaterial niedergeschlagen. Die thermische Behandlung erfolgt vorzugsweise unter Reaktionsbedingungen.

Man kann das Trägermaterial in einer Vakuumbedampfungsanlage diskontinuierlich oder kontinuierlich mit Silber bedampfen. Dazu wird das aufzubringende Silber im Vakuum vorzugsweise bei $10^{-4}$ bis $10^{-12}$ bar so stark erhitzt, daß es verdampft und sich auf dem Träger niederschlägt. Das Trägermaterial wird so angeordnet, daß ein möglichst großer Teil des Dampfstromes auf dem Träger kondensiert. Als Vakuumaufdampftechniken kommen alle bekannten Beschichtungsverfahren in Betracht, insbesondere die thermische Verdampfung. Man kann aber auch eine sogenannte Flash-Verdampfung, eine Kathodenzerstäubung sowie eine Kombination von thermischer Verdampfung und Kathodenzerstäubung durchführen. Die thermische Verdampfung kann durch direkte oder indirekte elektrische Heizung erfolgen. Auch die Elektronenstrahl-Verdampfung kann eingesetzt werden. Bei dieser Methode wird das Silber in einem Tiegel mittels eines Elektronenstrahls oberflächlich so stark erhitzt, daß es verdampft. Die Verdampfung kann in Gegenwart irgendeines Gases, insbesondere in Gegenwart von Inertgas oder Luft, erfolgen. Gezielte Zusätze geeigneter Mengen reaktiver Gase zum Restgas bewirken beim Schichtaufbau chemische Reaktionen, was beispielsweise zur Bildung von Oxiden, Nitriden oder Carbiden führen kann. Das zu bedampfende Trägermaterial wird über der Verdampfungsquelle angeordnet, so daß der Silberdampf darauf kondensiert.

Mit diesen Methoden kann man das Trägermaterial mit Verbindungen, die als Promotoren und Stabilisatoren wirken, gezielt und optimal dotieren. Man kann nach den oben genannten Methoden das Trägermaterial auch zunächst mit einer temperaturbeständigen Oxidschicht beschichten. Dazu wird entweder das entsprechende Oxid direkt aufgedampft oder man dampft das Metall auf und wandelt es anschließend durch Temperung bei Temperaturen im Bereich von etwa 300 bis 700°C an der Luft ins Oxid um. Die Oxidschicht kann aus Oxiden folgender Metalle bestehen: B, Al, Ba, Sr, Ca, Mg, Be, W, Mo, Ti, Sc, Y, La, Ce und anderen Seltenerdmetallen. Auf eine derart erzeugte thermisch stabile Unterlage wird dann das Silber als Aktivkomponente aufgedampft. und man erhält nach der thermischen Behandlung einen hochaktiven Silberkatalysator.

Wenn es sich beim Trägermaterial um Drahtgestricke handelt, beispielsweise um Raschigringe aus feinen Drähten, werden sie zweckmäßig in einen Drehtrommelkorb für Schüttgut gefüllt und darin bedampft.

Durch die angewendete Vakuumaufdampftechnik lassen sich Schichten beliebiger Dicke der Aktivkomponente und Promotoren bzw. Stabilisatoren, welche beispielsweise zur Verhinderung einer Rekristallisation dienen können, erzielen. Man kann beispielsweise dünne Schichten aufbringen, welche im Dickenbereich zwischen wenigen Angstrom und maximal 1 μm liegen. Man kann aber auch dickere Schichten aufbringen. Diese können beispielsweise im Bereich von 10 bis 10 000 nm liegen. Besonders bevorzugt sind Schichtdicken von 100 bis 1 000 nm.

Im Gegensatz zu den bisherigen Anwendungen der Aufdampftechnik in der Optik- und Elektroindustrie, bei denen hohe Reinheitsanforderungen an die Träger- und Aufdampfmaterialien gestellt werden, eine bestimmte Kondensationstemperatur am Träger und eine bestimmte Aufdampfrate eingestellt werden muß, da Fehlstellen in Aufdampffilmen die optischen, elektrischen und magnetischen Eigenschaften stark beeinflussen und hierfür ein erheblicher Aufwand zur reproduzierbaren Herstellung solcher Filme erforderlich ist, geht es beim erfindungsgemäßen Verfahren zur Herstellung der Trägerkatalysatoren darum, möglichst ungeordnete und gestörte, polykristalline Schichten herzustellen. Deshalb sind üblicherweise keine besonders guten Vakuumbedingungen erforderlich.

Nach dem erfindungsgemäßen Verfahren läßt sich der Katalysator systematisch aufbauen. Beispielsweise kann man zunächst den Träger mit einer Oxidschicht bedampfen und darauf die Aktivkomponente aufbringen. Auch lassen sich Aktivkomponente und Promotoren in mehreren Wechselschichten herstellen. Durch Einlaß eines reaktiven Gases in den Recipienten können Promotorschichten aus Oxiden oder anderen Verbindungen hergestellt werden. So kann man beispielsweise das Silber mit Halogeniden von Vanadium, Niob, Tantal, Chrom, Molybdän und Wolfram dotieren, insbesondere wenn der Katalysator zur Verwendung bei der Oxidation ungesättigter organischer Verbindungen, vorgesehen ist. Hierbei kommen insbesondere die Oxidation von Ethylen mit Sauerstoff unter Bildung von Ethylenoxid und die Oxidation von Propylen unter Bildung von Propylenoxid in Betracht.

Auch Tempervorgänge können zwischengeschaltet werden. Man hat so die Möglichkeit, alle Einzelschritte, die zum Aufbau eines komplexen Katalysatorsystems notwendig sind, in einem Arbeitsgang durchzuführen.

Man kann gewünschtenfalls durch wechselweises Aufdampfen von Aktivkomponente und strukturellen Promotoren die Aktivkomponente feinkristallin erhalten.

Das auf die vorstehende Weise hergestellte Katalysatormaterial, das beispielsweise in Form eines beschichteten Gewebes, Netzes oder einer Folie vorliegt, kann anschließend zu sogenannten Katalysatorpaketen geformt oder zusammengestellt werden. Hierbei handelt es sich um räumliche Anordnungen eines oder mehrerer oder einer Vielzahl von Trägerkatalysatorelementen in einer für den jeweiligen Anwendungszweck besonders geeigneten Form oder Packung.

Nach einer bevorzugten Ausführungsform werden Metallnetze aus hitzebeständigem Material mit Silber bedampft, anschließend getempert und daraus Netzscheiben hergestellt, die in Form eines Katalysatornetzpaketes in den Reaktor eingebaut werden. Dazu werden abwechselnd Katalysatornetzscheiben und Zwischenringe übereinandergelegt und so im Reaktor ein sogenanntes Katalysatornetzpaket hergestellt. Der Vorteil eines derartigen Katalysatornetzpakets ergibt sich durch einen Vergleich mit der DE-A-2 715 209. Dort wird die exotherme, oxidative Dehydrierung organischer Verbindungen an sehr kurzen Reaktionsschichten von 3,5 bis 5 cm Länge durchgeführt. Innerhalb dieser relativ kurzen Katalysatorschicht bildet sich eine Temperaturdifferenz $\Delta T$ von etwa 300 bis 400°C aus. Die mangelhafte Temperaturkontrolle und die ungleichmäßige Durchströmung einer derartigen Silbergranulatschüttung führt zu lokalen Übertemperaturen, auch "hotspots" genannt. Weiterhin sintern die einzelnen Katalysatorteilchen teilweise zusammen. Als Ergebnis erzielt man eine schlechte Selektivität. Demgegenüber gewährleisten die Katalysatornetzpakete ein einheitliches Strömungsverhalten in der Reaktionszone. Das Katalysatornetzpaket bietet außerdem die Möglichkeit zur exakten Messung des Temperaturprofils in der relativ kurzen Katalysatorschicht. Dies geschieht durch den Einbau von Thermoelementen zwischen den einzelnen Katalysatornetzen. Man erhält so das gesamte Temperaturprofil der Reaktionszone, was notwendige Voraussetzung für eine optimale Regelung der Eingangsmengen, sowie der Eingangstemperatur darstellt. Besitzt man exakte Information über die Temperatur an jeder Stelle in der Reaktionszone, so kann man durch gezielte Kühlmaßnahmen einen Teil der Reaktionswärme abführen und so eine Optimierung der Reaktionsführung erreichen.

Vergleichbare Ergebnisse lassen sich auch mit Katalysatorgitterpaketen oder anderen geometrischen Formen, z. B. Lochblech- oder Wabenformen, oder auch mit Katalysatorpakten auf Basis Trägerwolle, z. B. Metallträgerwolle erzielen.

Der erfindungsgemäße Trägerkatalysator läßt sich für eine Vielzahl von Oxidehydrierungen und Oxidationen einsetzen. So läßt sich Methanol mit Sauerstoff zu Formaldehyd umsetzen. Die Reaktion wird vorzugsweise bei Temperaturen zwischen 650 und 750°C, insbesondere etwa 700°C, durchgeführt. Die Umsetzung von Ethylenglykol mit Sauerstoff führt zur Bildung von Glyoxal. Hierbei arbeitet man vorzugsweise mit einem Trägerkatalysator, dessen Silberschicht mit Kupfer dotiert ist. Als Reaktionstemperatur eignet sich ein Bereich von 250 bis 400°C, insbesondere etwa 300°C.

Bei den Oxidationsreaktionen ist die Verwendung des Katalysators zur Bildung von Ethylenoxid oder Propylenoxid aus Ethylen bzw. Propylen besonders bevorzugt, wie vorstehend bereits erwähnt wurde.

Besonders geeignet ist der erfindungsgemäße Trägerkatalysator zur Herstellung von 3-Methyl-buten-1-alen durch oxidative Dehydrierung von 3-Methyl-buten-1-olen. Die weitere Erläuterung der Erfindung in den Beispielen erfolgt unter Bezugnahme auf diese Reaktion, und zwar im Vergleich zum Silbergranulatfestbettverfahren. Die Erfindung ist aber nicht auf diese Verwendung des Katalysators beschränkt.

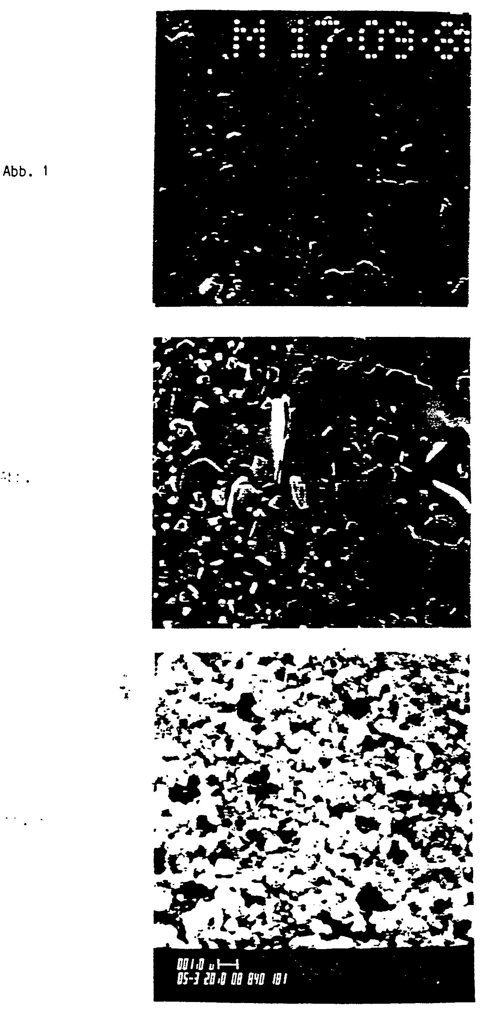

In den Zeichnungen sind in den Abbildungen 1 bis 3 elektronenmikroskopische Aufnahmen der Oberfläche des mit einer Silberschicht als Aktivkomponente versehenen erfindungsgemäßen Trägerkatalysators dargestellt.

Abb. 1 zeigt das mit der Silberschicht im Vakuum bedampfte Trägermaterial vor der thermischen Behandlung. Es ist zu ersehen, daß die Silberschicht einigermaßen glatt und ungestört ist. In dieser Phase ist sie noch nicht polykristallin. Der Trägerkatalysator ist noch nicht aktiv.

In Abb. 2 ist die Silberschicht nach der thermischen Behandlung dargestellt. Man erkennt den polykristallinen Aufbau der Silberschicht, die nunmehr auch eine wesentlich größere Oberfläche aufweist. Dieser Katalysator ist hochaktiv.

Abb. 3 zeigt die Silberschicht eines anderen erfindungsgemäßen Katalysators, welche durch thermische Behandlung unter Reaktionsbedingungen erhalten wurde. Auch hier fällt die große Oberflächenentwicklung auf. Der Katalysator ist hochaktiv.

Die nachfolgenden Beispiele dienen zur weiteren Erläuterung der Erfindung.

## Beispiel 1

Die Katalysatorherstellung erfolgte in einer Hochvakuum-Bedampfungsanlage. Mit dieser Vorrichtung lassen sich Metalle, wie z. B. Silber, mittels eines Elektronenstahls verdampfen und auf Trägermaterialien in Form polykristalliner Filme kondensieren. Dazu wird das zu bedampfende Trägernetz in dem Rezipienten der Anlage so angeordnet, daß der Silberdampfstrom das Trägermaterial gleichmäßig beschichtet.

Als Trägermaterial wurde ein hitzebeständiges Edestahlgewebe mit den Legierungsbestandteilen Cr und Al benutzt (Eigenschaftsangaben in DIN 17470). Rechteckige Netzstücke dieses Edelstahlgewebes wurden in einen Metallrahmen gespannt und über der Verdampfungsquelle angeordnet. Mittels einer Schwenkvorrichtung konnte der Rahmen unter Vakuum um 180°C gedreht werden, so daß das Gewebe beidseitig bedampft werden konnte. Das Metallgewebe wurde zunächst im Ultraschallbad gereinigt und nach dem Trocknen in die Bedampfungsanlage eingebaut. Der Rezipient wurde dann evakuiert und bei einem Restgasdruck von 1,3 · 10⁻⁶ mbar mittels eines Elektronenstahls Silber aus einem Tiegel verdampft und auf dem Trägernetz ein Silberfilm von 200 nm Schichtdicke kondensiert. Die Schichtdicke wurde mit einem Schwingquarz, der auch die Aufdampfrate steuerte, gemessen. Sobald das Trägernetz beidseitig beschichtet war, belüftete man die Apparatur.

Aus dem so gewonnenen Katalysatornetzgewebe stanzte man mit einem Stanzeisen runde Scheiben mit einem Durchmesser von 18 mm aus. Diese Katalysatornetzscheiben wurden in ein Reaktionsrohr senkrecht zur Strömungsrichtung eingebaut, und zwar derart, daß zwischen jede Netzscheibe ein Abstandsring von 0,5 mm gelegt wurde. Man erhielt dann mit 20 Katalysatornetzscheiben übereinander ein Katalysatornetzpaket von 25 mm Höhe.

Zur oxidativen Dehydrierung von 3-Methyl-buten-1-ol (MBE) verdampfte man ein Gemisch aus 85 Gew.% MBE und 15 Gew.-% $H_2O$ bei 150°C, vermischte mit vorgewärmter Luft, brachte mittels eines Vorheizers auf eine bestimmte Eingangstemperatur und leitete das Gemisch dann durch das Katalysatornetzpaket hindurch. Das gasförmige Reaktionsprodukt wurde im anschließenden Quench, mit Hilfe einer Kühlvorrichtung, die mit Kühlsole von 0°C gekühlt war, kondensiert und im ebenfalls gekühlten Abscheider gesammelt. Der gasförmige Anteil des Reaktionsproduktes gelangte über eine Trockeneiskühlung, mit der die Leichtsiederanteile kondensiert wurden, zur gaschromatographischen Analyse und dann über eine Gasuhr ins Abgas. Die vereinigten Kondensatmengen trennte man in eine organische und eine wäßrige Phase. Von beiden Phasen wurden gaschromatographische Analysen erstellt und der Wassergehalt ermittelt. Mit diesen Analysendaten wurden der Umsatz an MBE sowie die Selektivität berechnet. Die Umsetzung von MBE erfolgte an dem oben beschriebenen Katalysatornetzpaket, welches aus 20 Katalysatornetzscheiben bestand und eine Gesamthöhe von 25 mm besaß. Die Schichtdicke des Silberfilms betrug 200 nm. Unter folgenden Reaktionsbedingungen wurde die oxidative Dehydrierung von MBE durchgeführt:

Querschnittbelastung an MBE: $\left[\frac{74\,g\,MBE}{cm^2 \cdot h}\right]$

Luftmenge: $30,8 \left[\frac{Nl}{cm^2 \cdot h}\right]$

Reaktoreingangstemperatur: 290°C

(N = Normliter)

Am Katalysatorausgang wurde eine Temperatur von 445°C gemessen. Man erhielt bei einem Umsatz von 55 % eine Selektivität von 82 %. Die gebildete Menge an Wertprodukt pro Gramm eingesetztem Edelmetall betrug

$$3300 \left[\frac{g\,Wertprodukt}{g\,Silber \cdot h}\right]$$

**Beispiel 2**

Es wurden 25 Katalysatornetzscheiben mit einem Durchmesser von 18 mm, die in einer Schichtdicke von 200 nm Ag bedampft waren, zu einem Katalysatornetzpaket von 30 mm Höhe zusammengesetzt. Analog Beispiel 1 wurde die Umsetzung von MBE mit einer erhöhten Belastung von

$$113 \left[\frac{g\,MBE}{cm^2 \cdot h}\right]$$

durchgeführt.

Die Luftmenge pro Querschnittseinheit betrug

$$49,4 \left[\frac{Nl}{cm^2 \cdot h}\right]$$

und die Eingangstemperatur wurde auf 217°C erniedrigt. Bei einem Umsatz von 63 % erhielt man eine Selektivität von 81 % und damit

$$3800 \left[\frac{g\,Wertprodukt}{g\,Silber \cdot h}\right]$$

**Beispiel 3** (Vergleichsbeispiel)

In das Reaktionsrohr aus Beispiel 1 wurde anstelle des erfindungsgemäßen Katalysatornetzpaketes eine Silbergranulatschicht entsprechend der DE-A-2 715 209 eingebaut und die Umsetzung von MBE analog Beispiel 1 durchgeführt.

Die Querschnittsbelastung betrug

$$\left[\frac{69\ g\ MBE}{cm^2\ h}\right]$$

die Luftmenge pro Querschnittseinheit

$$27\left[\frac{Nl}{cm^2\ h}\right]$$

und die Reaktoreintrittstemperatur 259°C. Man erhielt einen Umsatz von 54 % bei einer Selektivität von 73 %. Die Menge an Wertprodukt pro Gramm Silber betrug

$$2,4\ \left[\frac{g\ Wertprodukt}{g\ Silber\cdot h}\right]$$

In der folgenden Tabelle sind die Daten der Beispiele 1 bis 3 nochmals dargestellt.

| Beispiel | Katalysator | Querschnitts-belastung $\frac{g\ MBE}{cm^2\cdot h}$ | Luftmenge pro Querschnitts-einheit $\frac{Nl}{cm^2\cdot h}$ | Reaktorein-trittstem-peratur [°C] | Umsatz [%] | Selekti-vität [%] | $\frac{Wertprodukt}{g\ Silber\cdot h}$ |
|---|---|---|---|---|---|---|---|
| 1 | 20 Katalysator-netzscheiben 200 nm Ag | 74 | 30,0 | 290 | 55 | 82 | 3300 |
| 2 | 25 Katalysator-netzscheiben 200 nm Ag | 113 | 49,4 | 217 | 63 | 81 | 3800 |
| 3 | 30 nm Silber-granulatschüttung | 69 | 27 | 259 | 54 | 73 | 2,4 |

**Patentansprüche**

1. Trägerkatalysator in Form eines mit einer Silberschicht als Aktivkomponente versehenen Trägermaterials, insbesondere eines netz-, gewebe-, oder folienartigen Trägermaterials, dadurch gekennzeichnet, daß die Silberschicht polykristallin ist und durch Bedampfen des Trägermaterials mit metallischem Silber und anschließende thermische Behandlung bei 200 bis 800°C gebildet wurde.

2. Trägerkatalysator nach Anspruch 1, dadurch gekennzeichnet, daß die thermische Behandlung unter Reaktionsbedingungen erfolgte.

3. Trägerkatalysator nach einem der Ansprüche 1 oder 2, dadurch gekennzeichnet, daß die thermische Behandlung bei 300 bis 500°C durchgeführt wurde.

4. Trägerkatalysator nach einem der Ansprüche 1 bis 3, dadurch gekennzeichnet, daß das Trägermaterial netzförmig ist.

5. Trägerkatalysator nach einem der Ansprüche 1 bis 4, dadurch gekennzeichnet, daß das Trägermaterial vor dem Aufdampfen der Silberschicht mit einer temperaturbeständigen Oxidzwischenschicht versehen wurde.

6. Trägerkatalysator nach einem der Ansprüche 1 bis 5, dadurch gekennzeichnet, daß das Trägermaterial ein Chrom/Aluminium-Edelstahlgewebe oder -netz ist.

7. Trägerkatalysator nach einem der Ansprüche 1 bis 6 in Form eines Katalysatorpakets.

8. Verfahren zur Herstellung des Trägerkatalysators nach einem der Ansprüche 1 bis 7, durch Aufbringen einer Silberschicht auf das Trägermaterial, dadurch gekennzeichnet, daß man die Silberschicht auf das Trägermaterial aufdampft und anschließend bei 200 bis 800°C eine thermische Behandlung durchführt.

9. Verfahren nach Anspruch 8, dadurch gekennzeichnet, daß man die thermische Behandlung unter Reaktionsbedingungen durchführt.

10. Verfahren nach einem der Ansprüche 8 oder 9, dadurch gekennzeichnet, daß man die thermische Behandlung bei einer Temperatur von 300 bis 500°C durchführt.

11. Verfahren nach einem der Ansprüche 8 bis 10, dadurch gekennzeichnet, daß man das aufzubringende Silber im Vakuum bei $10^{-1}$ bis $10^{-12}$ bar verdampft und auf das Trägermaterial niederschlägt.

12. Verfahren nach Anspruch 11, dadurch gekennzeichnet, daß man das aufzubringende Silber im Vakuum bei $10^{-6}$ bis $10^{-10}$ bar verdampft.

13. Verfahren nach einem der Ansprüche 8 bis 12, dadurch gekennzeichnet daß man vor dem Aufdampfen der Silberschicht auf dem Trägermaterial eine temperaturbeständig Metalloxidschicht als Zwischenschicht bildet.

14. Verwendung des Trägerkatalysators nach einem der Ansprüche 1 bis 7 bei der oxidativen Dehydrierung organischer Verbindungen.

15. Verwendung nach Anspruch 14 zur Herstellung von 3-Methyl-buten-1-alen durch oxidative Dehydrierung von 3-Methylbuten-1-olen.

16. Verwendung des Trägerkatalysators nach einem der Ansprüche 1 bis 7 bei der Oxidation ungesättigter organischer Verbindungen.

## Claims

1. A supported catalyst in the form of a carrier provided with a silver layer as the active component, in particular a net-like, fabric-like or sheet-like carrier, wherein the silver layer is polycrystalline and is formed by coating the carrier with metallic silver by vapor deposition and then subjecting it to a heat treatment at from 200 to 800°C.

2. A supported catalyst as claimed in claim 1, wherein the heat treatment is carried out under reaction conditions.

3. A supported catalyst as claimed in either of claims 1 and 2, wherein the heat treatment is carried out at from 300 to 500°C.

4. A supported catalyst as claimed in any of claims 1 to 3, wherein the carrier is net-like.

5. A supported catalyst as claimed in any of claims 1 to 4, wherein the carrier is provided with an intermediate heat-resistant oxide layer prior to application of the silver layer by vapor deposition.

6. A supported catalyst as claimed in any of claims 1 to 5, wherein the carrier is a chromium-aluminum stainless steel fabric or net.

7. A supported catalyst as claimed in any of claims 1 to 6, in the form of a catalyst packet.

8. A process for the preparation of a supported catalyst as claimed in any of claims 1 to 7, by applying a silver layer onto the carrier, wherein the silver layer is applied onto the carrier by vapor deposition and a heat treatment is then carried out at from 200 to 800°C.

9. A process as claimed in claim 8, wherein the heat treatment is carried out under reaction conditions.

10. A process as claimed in either of claims 8 and 9, wherein the heat treatment is carried out at from 300 to 500°C.

11. A process as claimed in any of claims 8 to 10, wherein the silver to be applied is vaporized under reduced pressure of from $10^{-1}$ to $10^{-12}$ bar and applied onto the carrier.

12. A process as claimed in claim 11, wherein the silver to be applied is vaporized under reduced pressure of from $10^{-6}$ to $10^{-10}$ bar.

13. A process as claimed in any of claims 8 to 12, wherein a heat-resistant metal oxide layer is formed as an intermediate layer prior to application of the silver layer on the carrier by vapor deposition.

14. Use of a supported catalyst as claimed in any of claims 1 to 7 in the oxidative dehydrogenation of organic compounds.

15. Use as claimed in claim 14 for the preparation of a 3-methylbuten-1-al by oxidative dehydrogenation of a 3-methylbuten-1-ol.

16. Use of a supported catalyst as claimed in any of claims 1 to 7 in the oxidation of unsaturated organic compounds.

## Revendications

1. Catalyseur sur support sous forme d'un matériau support pourvu d'une couche d'argent comme composant actif, en particulier un matériau support constitué d'une toile métallique, d'un tissu ou d'une feuille mince, caractérisé en ce que la couche d'argent est polycristalline et en ce qu'elle a été formée, par un dépôt d'argent métallique vaporisé sur le matériau support et par un traitement thermique subséquent du matériau à une température de 200 à 800°C.

2. Catalyseur sur support suivant la revendication 1, caractérisé en ce que le traitement thermique a eu lieu dans les conditions de la réaction.

3. Catalyseur sur support suivant la revendication 1 ou 2, caractérisé en ce que le traitement thermique a été effectué à une température de 300 à 500°C.

4. Catalyseur sur support suivant l'une quelconque des revendications 1 à 3, caractérisé en ce que le matériau support est sous forme d'une toile métallique.

5. Catalyseur sur support suivant l'une quelconque des revendications 1 à 4, caractérisé en ce que le matériau support a été pourvu, avant le dépôt de la couche d'argent par vaporisation, d'une couche intermédiaire d'un oxyde stable à la chaleur.

6. Catalyseur sur support suivant l'une quelconque des revendications 1 à 5, caractérisé en ce que le matériau support est un tissu ou une toile métallique en acier spécial chrome/aluminium.

7. Catalyseur sur support suivant l'une quelconque des revendications 1 à 6 sous forme d'un paquet de catalyseurs.

8. Procédé de fabrication du catalyseur sur support suivant l'une quelconque des revendications 1 à 7, suivant lequel on applique une couche d'argent sur le matériau support, caractérisé en ce que l'on dépose la couche d'argent par vaporisation sur le matériau support et en ce que l'on effectue ensuite un traitement thermique à une température de 200 à 800°C.

9. Procédé suivant la revendication 8, caractérisé en ce que l'on effectue le traitement thermique dans les conditions de la réaction.

10. Procédé suivant la revendication 8 ou 9, caractérisé en ce que l'on effectue le traitement thermique à une température de 300 à 500°C.

11. Procédé suivant l'une quelconque des revendications 8 à 10, caractérisé en ce que l'on vaporise l'argent à déposer dans un vide de $10^{-1}$ à $10^{-12}$ bar.

12. Procédé suivant la revendication 11, caractérisé en ce que l'on vaporise l'argent à déposer dans un vide de $10^{-6}$ à $10^{-10}$ bar.

13. Procédé suivant l'une quelconque des revendications 8 à 12, caractérisé en ce que l'on forme, avant le dépôt de la couche d'argent par vaporisation sur le matériau support, une couche d'oxyde métallique stable à la chaleur comme couche intermédiaire.

14. Utilisation du catalyseur sur support suivant l'une quelconque des revendications 1 à 7 dans la déshydrogénation par oxydation des composés organiques.

15. Utilisation suivant la revendication 14 pour la préparation de méthyl-3-buténals-1 par déshydrogénation par oxydation de méthyl-3-buténols-1.

16. Utilisation du catalyseur sur support suivant l'une quelconque des revendications 1 à 7 dans l'oxydation de composés organiques non saturés.

Abb. 1